# FASCICULE DE BREVET EUROPEEN

(11) **EP 3 110 425 B1**
(45) Date de publication et mention de la délivrance du brevet: **13.11.2019**
(21) Numéro de dépôt: 15713215.0
(22) Date de dépôt: 02.03.2015
(51) Int. Cl.: A61K 31/7008, A61K 31/7016, A61P 27/04, A61K 9/00

(54) **COMPOSITION OPHTALMIQUE RÉMANENTE, NOTAMMENT POUR LE TRAITEMENT DE LA SÉCHERESSE OCULAIRE**
NICHTFLÜCHTIGE OPHTHALMISCHE ZUSAMMENSETZUNG, INSBESONDERE ZUR BEHANDLUNG VON TROCKENEM AUGE
NON-VOLATILE OPHTHALMIC COMPOSITION, IN PARTICULAR FOR TREATING DRY EYE SYNDROME

(30) Priorité: 28.02.2014 FR 1451662
(43) Date de publication de la demande: 04.01.2017
(73) Titulaire: Laboratoires Théa, 63100 Clermont-Ferrand (FR); Medical University Of Vienna, 1090 Vienna (AT)
(72) Inventeur: MERCIER, Fabrice, F-63000 Clermont-Ferrand (FR); SCHMETTERER, Léopold, A-1120 Vienna (AT)
(74) Mandataire: Cabinet Laurent & Charras
(86) Numéro de dépôt international: PCT/FR2015/050499
(87) Numéro de publication internationale: WO 2015/136186

(56) Documents cités:
- EP-A1- 1 598 371
- WO-A1-2008/077712
- WO-A1-2012/046009
- WO-A2-2008/049043
- CN-A- 1 651 090
- CN-A- 101 691 478
- CN-B- 101 691 479
- US-A1- 2006 222 623
- US-A1- 2011 319 502
- WAN XIUYU ET AL: "Preparation of trehalose eye drops containing sodium hyaluronate", ZHONGGUO YAOXUE ZAZHI - CHINESE PHARMACEUTICAL JOURNAL, ZHONGGUO YAOXUEHUI ZHUBAN CHUBAN. ZHONGGUO-YAOXUE-ZAZHI BIANJI WEIYUANHUI, CN, vol. 43, no. 11, 1 janvier 2008 (2008-01-01), pages 838-840, XP008173169, ISSN: 1001-2494
- HARMON PATRICIA S ET AL: "Detailed characterization of hyaluronan using aqueous size exclusion chromatography with triple detection and multiangle light scattering detection.", JOURNAL OF BIOMEDICAL MATERIALS RESEARCH. PART B, APPLIED BIOMATERIALS OCT 2012, vol. 100, no. 7, octobre 2012 (2012-10), pages 1955-1960, XP002739531, ISSN: 1552-4981
- DATABASE MEDLINE [Online] US NATIONAL LIBRARY OF MEDICINE (NLM), BETHESDA, MD, US; mars 1997 (1997-03), AVISAR R ET AL: "Comparative study of tear substitutes and their immediate effect on the precorneal tear film.", XP002739532, Database accession no. NLM9313790 & AVISAR R ET AL: "Comparative study of tear substitutes and their immediate effect on the precorneal tear film.", ISRAEL JOURNAL OF MEDICAL SCIENCES MAR 1997, vol. 33, no. 3, mars 1997 (1997-03), pages 194-197, ISSN: 0021-2180
- DATABASE MEDLINE [Online] US NATIONAL LIBRARY OF MEDICINE (NLM), BETHESDA, MD, US; juillet 1992 (1992-07), SNIBSON G R ET AL: "Ocular surface residence times of artificial tear solutions.", XP002739533, Database accession no. NLM1424647

## Description

### DOMAINE DE L'INVENTION

La présente invention propose une composition ophtalmique pour application topique, destinée notamment à traiter la sécheresse oculaire. Une telle composition comprend de l'acide hyaluronique ou un de ses sels de poids moléculaire compris entre 100 et 800 kDa, auquel est associé un oligosaccharide. De manière surprenante, la présente demande rapporte une meilleure rémanence d'une composition basée sur une telle association.

### ETAT DE LA TECHNIQUE

Le syndrome de l'œil sec, également dénommé sécheresse oculaire ou kérato-conjonctivite sèche (KCS), est une pathologie pour laquelle la quantité de larmes produites par les glandes lacrymales est insuffisante. Il en résulte un inconfort au niveau de l'œil, qui s'accompagne de démangeaisons, picotements et/ou sensations de brûlures.

La sécheresse oculaire peut résulter d'une anomalie des glandes lacrymales, d'une inflammation des paupières, d'une inflammation oculaire due à une allergie, d'une chirurgie réfractive (notamment au laser), d'une carence en certains lipides dans l'alimentation quotidienne, d'un port prolongé de lentilles de contact, d'une altération hormonale, d'une maladie auto-immune, ou d'effets secondaires de certains médicaments.

Dans le traitement de la sécheresse oculaire, l'application de larmes artificielles, également appelées gouttes ophtalmiques lubrifiantes ou humectantes, apporte un soulagement local mais de courte durée, sans solutionner le problème d'un point de vue systémique : elles n'aident en rien l'organisme à améliorer la quantité ou la qualité des larmes et ne constituent qu'une solution de substitution temporaire. Les larmes artificielles de viscosité faible à moyenne sont généralement à base d'alcools polyvinyliques ou de dérivés cellulosiques, alors que celles à viscosité plus élevée contiennent des carbomères ou de l'acide hyaluronique.

Ainsi, l'acide hyaluronique est aujourd'hui largement utilisé dans le cadre de formulation topique destinée au traitement de la sécheresse oculaire.

L'acide hyaluronique est utilisé en collyre pour l'hydratation de la cornée, où son effet "barrière" aux frottements atténue la douleur provoquer par la sècheresse oculaire. Il forme, à la surface de l'œil, un film transparent, lubrifiant et mouillant, protégeant la cornée contre le dessèchement. Ces propriétés permettent un soulagement efficace des signes fonctionnels et une réduction du nombre d'instillations quotidiennes pour le patient.

Ainsi, de nombreux malades ne sont pas soulagés par les simples larmes artificielles ou le sérum physiologique que les pharmaciens ou certains médecins conseillent. Trop secs, leurs yeux ont besoin d'une substance plus efficace, qui fonctionne plus longtemps qu'une dizaine de minutes. Dans la plupart des cas, on leur conseille alors des collyres à base d'acide hyaluronique.

L'acide hyaluronique est une substance naturelle, déjà présente dans le corps. Très visqueuse, elle permet, dans le corps, d'augmenter le « glissement » des articulations entre elles. C'est donc en toute logique que les laboratoires ont utilisé ce produit pour créer des collyres plus efficaces que les gouttes classiques. Les études montrent que ces collyres sont très bien acceptés par les yeux les plus secs. Après instillation, une légère gêne de quelques secondes à quelques minutes peut apparaitre. Un léger flou au niveau de la vision prend place. Il vaut donc mieux ne pas utiliser le produit « en urgence », avant de prendre la route. La gêne disparait très vite et le produit reste en place beaucoup plus longtemps qu'un collyre simple. Le temps d'action dépend de la sécheresse des yeux. En règle générale, là où un collyre simple a un effet pendant 10 à 15 minutes, l'action de l'acide hyaluronique dure plutôt de 45 minutes à 1h.

En conclusion, les collyres à base d'acide hyaluronique sont efficaces pour une sécheresse oculaire légère à modérée. Les malades d'une sécheresse grave peuvent également y trouver un intérêt, mais doivent en général se tourner vers des produits différents, comme des collyres à base d'immunosuppresseur tel que la ciclosporine.

En rapport avec l'acide hyaluronique se pose toutefois le problème de son temps d'action limité et la nécessité pour le patient de répéter les instillations environ toutes les heures.

Pour améliorer l'efficacité de ce produit, il a été tenté de jouer sur sa concentration ou son poids moléculaire. En effet, il est admis que, plus l'acide hyaluronique est concentré et/ou de haut poids moléculaire, plus sa durée d'action est longue. Toutefois, il est constaté qu'une augmentation de la concentration et/ou du poids moléculaire se traduit par une augmentation de la viscosité de la composition contenant l'acide hyaluronique, ce qui se traduit, pour le patient, par un sentiment d'inconfort et/ou de gêne à l'instillation.

A noter que l'utilisation dans une composition ophtalmique d'acide hyaluronique réticulé, qui *a priori* est la forme la plus stable et la plus persistante, n'est pas autorisée et ne serait pas acceptable : en raison de sa viscosité élevée, son instillation serait douloureuse et entrainerait une forte gêne visuelle.

Le document CN 1 651 090 vise une matrice à base de tréhalose et d'acide hyaluronique pour la délivrance topique de molécules actives dans le domaine de l'ophtalmologie et démontre l'efficacité thérapeutique à 4 semaines d'une composition conservée.

Le document WAN et al. (Chin. Pharm. J., 43(11), 2008, pp 838-840) décrit des gouttes oculaires conservées contenant du tréhalose et de l'hyaluronate de sodium pour traiter l'ophtalmoxérose.

Le document CN 101 691 478 décrit une solution d'entretien de lentilles comprenant du tréhalose, possiblement de l'hyaluronate de sodium en tant qu'agent lubrifiant, et un agent antimicrobien.

Le document CN 101 691 479 décrit une solution d'entretien de lentilles comprenant du tréhalose et possiblement de l'hyaluronate de sodium en tant qu'agent lubrifiant, et rapporte un plus grand confort pour l'utilisateur de lentilles ainsi prétraitées.

Il existe donc un besoin évident de développer des formulations ophtalmiques à base d'un polymère lubrifiant et hydratant tel que l'acide hyaluronique, présentant une meilleure rémanence permettant à l'utilisateur d'espacer les applications, sans provoquer de gêne pendant ou après l'instillation.

### DESCRIPTION DE L'INVENTION

C'est dans ce cadre que se situe la présente invention. En effet et de manière surprenante, le Demandeur a constaté que l'ajout d'un oligosaccharide permettait d'augmenter la durée d'action et donc l'efficacité du polymère lubrifiant en présence, à savoir de l'acide hyaluronique ou un de ses sels de poids moléculaire compris entre 100 et 800 kDa.

Sans vouloir être lié à une quelconque théorie, il est supposé qu'une réticulation pourrait avoir lieu *in vivo* entre le polymère lubrifiant et l'oligosaccharide, augmentant ainsi la stabilité du polymère sans provoquer de gêne puisque cette réticulation se ferait *in situ,* c'est à dire post-instillation et éventuellement de manière graduelle. Cette hypothèse implique la présence *in vivo,* plus précisément au niveau de la surface oculaire, d'activateurs de la réticulation. Il pourrait s'agir des radicaux libres naturellement générés, présents en quantité encore plus importante dans le cas d'une inflammation.

Ainsi et selon un premier aspect, la présente demande décrit une composition ophtalmique comprenant un polymère lubrifiant et un oligosaccharide. Selon l'invention, la composition comprend de l'acide hyaluronique, ou un de ses sels, de poids moléculaire compris entre 100 et 800 kDa et un oligosaccharide.

Dans le cadre de la demande, un polymère lubrifiant, avantageusement hydratant, est défini comme étant capable de former un film transparent à la surface oculaire, notamment en raison de sa capacité de rétention de l'eau.

De manière avantageuse, ledit polymère est un polysaccharide, c'est-à-dire un polymère constitué de plusieurs oses liés entre eux par des liaisons O-osidiques. Encore plus avantageusement, il s'agit d'un glycosaminoglycane noté GAG.

Par ailleurs et de manière privilégiée, le polymère lubrifiant est un composé d'origine naturelle et non synthétique.

Selon un autre mode de réalisation, ce polymère n'est pas un dérivé de cellulose, en particulier ce polymère n'est pas de la carboxyméthylcellulose (CMC).

Les GAGs ont longtemps été désignés sous le terme de « mucopolysaccharides acides » en raison de leur forte capacité de rétention de l'eau (« muco »), de leur nature glucidique (« polysaccharides ») et de leur caractère acide provenant de leurs multiples charges négatives acides. Il s'agit en effet de chaînes linéaires (polymères non ramifiées) sulfatés (sauf l'acide hyaluronique) composées de la répétition d'un diholoside de base contenant toujours une hexosamine (glucosamine (GlcN) ou galactosamine (GalN)) et un autre ose (acide glucuronique (GlcA), acide iduronique (IdoA), galactose (Gal)). La glucosamine est soit N-sulfatée (GlcNS), soit N-acétylée (GlcNac). La galactosamine est toujours N-acétylée (GalNac).

Selon un mode de réalisation particulier, ledit glycosaminoglycane est choisi dans le groupe comprenant l'acide hyaluronique, le chondroïtine sulfate, le dermatane sulfate, le kératane sulfate, l'héparane sulfate (ou héparine), ou des sels pharmaceutiquement acceptable de ceux-ci.

L'acide hyaluronique, qui se caractérise par l'absence de O-sulfatation et un poids moléculaire élevé, est utilisé dans l'invention, notamment sous forme de hyaluronate de sodium.

Le polymère utilisé va de fait déterminer la viscosité de la composition ophtalmique. Cette viscosité étant dépendante à la fois de la concentration en polymère et du poids moléculaire de ce polymère, ces deux facteurs sont ajustés pour que la composition présente une viscosité adaptée ne causant pas de gêne (au moment de l'instillation ou après) à l'utilisateur.

Ainsi et de manière adaptée, notamment pour l'acide hyaluronique, la concentration centésimale de la composition en polymère lubrifiant est inférieure ou égale à 0,5%, avantageusement à 0,2%. En d'autres termes, le poids du polymère lubrifiant représente avantageusement au plus 0,5 g, voire 0,2 g dans 100 ml de la composition. Par ailleurs et dans un but d'efficacité, la concentration centésimale en polymère lubrifiant est avantageusement supérieure ou égale à 0,05% (0,05g/100ml), voire 0,1% (0,lg/100ml). En d'autres termes et selon un mode de réalisation privilégié, le polymère lubrifiant représente entre 0,05 et 0,5 g pour 100 ml de la composition, avantageusement entre 0,1 et 0,2 g/100 ml, par exemple 0,15 g/100 ml.

Comme déjà mentionné, le polymère est préférentiellement sous forme non réticulée. De surcroît, pour l'acide hyaluronique, un poids moléculaire (MW pour *« Molecular Weight* ») requis est compris entre 100 kDa et 800 kDa (medium MW).

L'autre composant de la formulation proposée dans le cadre de la présente invention est un oligosaccharide. Les oligosaccharides, également nommés oligoholosides ou oligosides, sont des oligomères formés d'un nombre n d'unités ose (ou monosaccharide), avec n généralement compris entre 2 et 10. Les oligosides peuvent être linéaires, ramifiés ou cycliques.

Il ne s'agit donc pas d'un polyol de type glycérol ou glycérine.

Selon un mode de réalisation particulier, l'oligosaccharide est un diholoside, qui peut être constitué de deux oses identiques (homo) ou différents (hétéro). Un exemple non limitatif est le rutinose (rhamnose α(1-6) glucose). De manière non limitative, d'autres oligosaccharides d'intérêt sont le tréhalose, le kojibiose, le nigerose, le maltose, l'isomaltose, le sophorose le laminaribiose, le cellobiose et le gentibiose, voire le tréhalulose, le saccharose, le turanose, le maltulose, le leucrose, l'isomaltulose, le gentiobiose, le mélibiose, le lactulose, le lactose, le rutinose, l'inulobiose, l'2alpha-Mannobiose, l'3alpha-Mannobiose.

A noter que ces oligosaccharides peuvent présenter des propriétés intrinsèques d'intérêt dans le domaine ophtalmique, en particulier une activité anti-inflammatoire, anti-apoptotique, ou de régulation de l'osmolarité et donc de captation de l'eau.

Selon un autre mode de réalisation, la concentration centésimale de la composition en oligosaccharide est inférieure ou égale à 5% en poids de la composition, avantageusement à 4%. En d'autres termes, le poids de l'oligosaccharide représente avantageusement au plus 5 g, voire 4 g dans 100 ml de la composition. Par ailleurs et dans un but d'efficacité, la concentration centésimale en oligosaccharide est avantageusement supérieure ou égale à 0,5% (0,5 g/100 ml), voire 2% (2 g/100 ml). En d'autres termes et selon un mode de réalisation privilégié, l'oligosaccharide représente entre 0,5 et 5 g pour 100 ml de la composition, avantageusement entre 2 et 4 g/100 ml, par exemple 3 g/100 ml.

De manière avantageuse, la composition selon l'invention présente une viscosité adaptée à l'application topique ophtalmique visée.

Ainsi, la composition selon l'invention présente préférentiellement une viscosité inférieure ou égale à 50 mPa.s (cP), voire inférieure ou égale à 20, 15, 10 ou même 5 mPa.s. De manière adaptée, elle est comprise entre 2 et 50 mPa.s, avantageusement comprise entre 2 et 15 mPa.s, encore plus avantageusement comprise entre 2 et 10 mPa.s, par exemple de l'ordre de 5 mPa.s. Ces valeurs correspondent aux valeurs mesurées à l'aide d'un viscosimètre à mobile tournant BROOKFIELD RVDV III à 25°C.

En pratique, cette viscosité est avantageusement proche de la viscosité des larmes humaines, évaluée à environ 6,5 mPa.s à 20°C et à gradient de vitesse proche de zéro. L'application de la solution selon l'invention ne provoque ainsi aucune sensation de gêne ou d'irritation. En effet, il a été déterminé qu'une élimination aisée et non gênante n'est possible que si la viscosité du mélange solution ophtalmique / larme est inférieure à 50 mPa.s.

Bien entendu, la composition selon l'invention peut contenir en outre un additif choisi dans le groupe des agents isotonisants de type non ioniques, des agents antioxydants et/ou des systèmes tampons.

De manière préférée, la solution selon l'invention présente un pH neutre, avantageusement compris entre 6,5 et 7,5.

Selon un autre mode de réalisation particulier, elle correspond à une formulation hypotonique à isotonique, avec une osmolalité avantageusement comprise entre 170 et 350 mosmol/kg. Cette caractéristique peut être conférée, par exemple, par ajout de chlorure de sodium (NaCl). L'oligosaccharide, à la concentration utilisée, peut également contribuer à la régulation de la tonicité de la solution.

Par ailleurs, la composition selon l'invention peut également contenir un ou plusieurs actifs, en quantité thérapeutiquement efficace. Il peut s'agir par exemple d'antibiotiques, d'antibactériens, d'antifongiques, d'antiviraux, d'immunosuppresseurs, d'anti-inflammatoires ou d'anti-glaucomateux tels que des prostaglandines, des béta-bloquants, des inhibiteurs de l'anhydrase carbonique ou encore des agonistes alpha-adrénergiques.

Un tel actif peut notamment être choisi dans le groupe suivant : aceclidine, acetazolamide, aciclovir, anecortave, apraclonidine, atropine, azapentacene, azelastine, bacitracine, befunolol, betamethasone, betaxolol, bimatoprost, brimonidine, brinzolamide, carbachol, carteolol, celecoxib, chloramphenicol, chlortetracycline, ciprofloxacin, cromoglycate, , cyclopentolate, cyclosporine, dapiprazole, demecarium, dexamethasone, diclofenac, dichlorphenamide, dipivefrine, dorzolamide, echothiophale, emedastine, epinastine, epinephrine, erythromycine, ethoxzolamide, eucatropine, fludrocortisone, fluorometholone, flurbiprofen, fomivirsen, framycetine, ganciclovir, gatifloxacine, gentamycine, homatropine, hydrocortisone, idoxuridine, indomethacine, isoflurophate, ketorolac, ketotifen, latanoprost, levobetaxolol, levobunolol, levocabastine, levofloxacine, lodoxamide, loteprednol, medrysone, methazolamide, metipranolol, moxifloxacine, naphazoline, natamycine, nedocromil, neomycine, norfloxacine, ofloxacine, olopatadine, oxymetazoline, pemirolast, pegaptanib, phenylephrine, physostigmine, pilocarpine, pindolol, pirenoxine, polymyxin B, prednisolone, proparacaine, ranibizumab, rimexolone, scopolamine, sezolamide, squalamine, sulfacetamide, suprofen, tetracaine, tetracyclin, tetrahydrozoline, tetryzoline, timolol, tobramycine, travoprost, triamcinulone, trifluoromethazolarnide, trifluridine, trimethoprime, tropicamide, unoprostone, vidarbine, xylometazoline, des sels pharmaceutiquement acceptables, ou des combinaisons de ceux-ci.

Comme mentionné précédemment, une composition selon l'invention persiste plus longtemps à la surface de l'œil. En présence d'un actif dans cette composition, il peut donc être envisagé de diminuer la concentration de cet actif, compensée par un temps d'action prolongé, et ainsi de potentiellement réduire les effets secondaires liés à cet actif. L'association polymère/oligosaccharide décrite dans le cadre de la présente demande, en raison de sa rémanence, permet donc de formuler des préparations à libération prolongée et d'améliorer la biodisponibilité du principe actif en présence.

Selon un mode de réalisation particulier, la composition selon l'invention est dépourvue de tout actif thérapeutique ophtalmique, tel que notamment ceux listés ci-dessus, autre que l'oligosaccharide et le polymère lubrifiant définis dans le cadre de l'invention.

Ainsi, une telle composition est utile dans le traitement de la sécheresse oculaire, en augmentant la rémanence du polymère lubrifiant, en particulier l'acide hyaluronique déjà connu pour son utilisation dans cette application.

Selon un autre mode de réalisation, la composition selon l'invention est dépourvue d'agent conservateur de type antimicrobien, notamment de type ammonium quaternaire, en particulier le chlorure de benzalkonium (BAK) connu pour ces effets délétères sur la surface oculaires. En d'autres termes, il s'agit avantageusement d'une composition dite non conservée.

En effet et de manière favorable, une composition selon l'invention, dépourvue de conservateur et notamment de BAK, présente une tension superficielle proche de celle de l'eau, de l'ordre de 70 mN/m. Ainsi, alors que l'eau purifiée a une tension superficielle égale à 73 mN/m, une composition selon l'invention possède avantageusement une tension superficielle comprise entre 60 et 75 mN/m, avantageusement supérieure ou égale à 60 voire 65 mN/m et inférieure ou égale à 75 voire 70 mN/m. A titre d'exemple, une composition contenant du BAK a une tension superficielle inférieure à 40 mN/m.

Dans le cadre de l'invention, par « agent conservateur de type anti-microbien ou anti-microbien », on désigne un agent conservateur présentant des propriétés anti-microbiennes, c'est-à-dire un composé capable de garantir la protection de la solution ophtalmique vis-à-vis d'une éventuelle contamination microbienne.

Des composés ammonium quaternaires, en particulier le chlorure de benzalkonium ou le polyquad, éventuellement en association avec l'EDTA, sont généralement utilisés en tant que conservateurs.

D'autres conservateurs sont par exemple :
- les conservateurs à base de guanidine (PHMB, chlorhexidine) ;
- l'alcool benzylique ;
- les parabènes (méthyl, éthyl, propyl, ...) ;
- les conservateurs à base de mercure, tels que le thimérosal ;
- le chlorobutanol ;
- le chlorure de benzéthonium ;
- les conservateurs oxydants (Purite, ...).

Notamment dans le cas d'une composition non conservée, la formulation de l'invention est avantageusement présentée dans des flacons à usage unique (unidose) ou dans un flacon multi-doses du type par exemple Abak® ou Comod® ou équivalent, de tels flacons permettant de délivrer des collyres sans conservateurs pendant plusieurs jours.

L'invention a donc également pour objet un flacon à usage unique (unidose) ou multidose adapté à des compositions non conservées comprenant la solution ophtalmique précédemment décrite, par exemple réalisé en PEBD, avantageusement de qualité EP ne contenant pas d'additifs.

Un autre objet de l'invention concerne l'utilisation de cette composition dans le traitement des troubles et pathologies oculaires.

Ainsi, l'association polymère lubrifiant et oligosaccharide peut être mis en œuvre dans le cadre de la sécheresse oculaire (ou syndrome de l'œil sec), dans ses formes modérées à sévères, et éventuellement pour le traitement des inflammations oculaires.

Dans le cas où la composition contient, outre cette association, un actif, la pathologie susceptible d'être traitée par cet actif peut être visée, notamment les infections oculaires, les allergies oculaires et le glaucome.

Une composition selon l'invention peut aussi bien être utilisée chez l'homme que chez l'animal.

Selon un mode de réalisation privilégié, cette composition est destinée à une application topique, avantageusement par instillation au niveau de l'œil.

La posologie est adaptée à la gravité de la pathologie mais consiste généralement à administrer dans l'œil affecté, une à plusieurs gouttes par jour. Comme démontré dans le cadre de la présente demande, la composition de la formulation proposée permet d'espacer les administrations, en comparaison notamment avec une composition ne contenant pas l'oligosaccharide. Ainsi, une posologie de moins de 5 gouttes par jour et par œil, voire moins de 4, de 3, de 2 ou même de 1 seule goutte par jour et par œil peut être envisagée.

Ainsi et selon un autre aspect, l'invention concerne l'utilisation d'un oligosaccharide pour augmenter la rémanence d'une composition ophtalmique, avantageusement topique (pour instillation dans l'œil), comprenant un polymère lubrifiant.

De manière générale, la rémanence (ou persistance) d'une composition est définie comme la durée pendant laquelle ladite composition continue à exercer son action, en l'absence de répétition de l'application.

Ainsi et de manière avantageuse, une composition selon l'invention, associant un polymère lubrifiant et un oligosaccharide, présente une rémanence ou persistance au moins 2 fois supérieure, voire 3 fois, voire 4 fois, voie même 5 fois supérieure à celle d'une composition comprenant uniquement le polymère lubrifiant (composition par ailleurs identique mais dépourvue d'oligosaccharide).

Dans la cadre de l'invention, la rémanence d'une composition peut être évaluée par mesure de l'épaisseur du film lacrymal, avantageusement chez des patients présentant un syndrome de sécheresse oculaire modéré (DES pour *« Dry Eye Syndrom* »). L'épaisseur du film lacrymal (TFT pour « *Tear Film Thickness* ») est mesurée par tomographie à cohérence optique de haute résolution (OCT pour « *Optical Coherence Tomography »*). Le protocole est avantageusement celui mis en œuvre dans les exemples de réalisation.

Ainsi, pour une efficacité (délai et intensité de l'augmentation) comparable sur la TFT, une composition selon l'invention présente une durée d'action prolongée par rapport à une composition en tout point identique mais dépourvue d'oligosaccharide, d'au moins un facteur 2, 3, 4 voire même 5 ou plus.

En d'autres termes, à un temps donné après instillation allant avantageusement de 30 minutes à 4 heures, l'efficacité (avantageusement estimée par mesure de la TFT) de l'association polymère lubrifiant et oligosaccharide est supérieure à celle du polymère lubrifiant seul. En pratique, ce prolongement de l'efficacité permet d'espacer les prises, en l'occurrence les instillations.

En pratique et comme démontré dans le cadre de la présente demande, l'ajout de l'oligosaccharide au polymère lubrifiant présente un avantage certain dans le cadre de la sécheresse oculaire.

Selon un autre aspect, la présente demande rapporte que l'association ou combinaison entre un polymère lubrifiant et un oligosaccharide constitue une base pour formuler des préparations à libération prolongée (« drug delivery system »), permettant d'améliorer la biodisponibilité, la rémanence et donc l'efficacité d'un actif, notamment tel que décrit précédemment.

Ainsi, la présente demande décrit également l'utilisation de la combinaison d'un polymère lubrifiant et d'un oligosaccharide pour augmenter la rémanence d'une composition ophtalmique, avantageusement topique.

Dans des modes de réalisation privilégiés, le polymère et l'oligosaccharide, aussi bien dans leur nature que dans leur concentration, sont comme décrits ci-dessus. Il en est de même pour la composition, aussi bien dans ses ingrédients que dans son application.

L'amélioration de la rémanence démontrée dans le cadre de la présente demande a pour conséquence directe une diminution possible de la fréquence des prises. Une autre conséquence, en particulier lorsque la composition contient en outre un actif, est une augmentation de l'efficacité de cet actif de par sa plus grande rémanence et donc la possibilité de diminuer sa concentration et ainsi les effets secondaires associés.

### EXEMPLES DE REALISATION

L'invention et les avantages qui en découlent ressortiront mieux à la lecture des exemples suivants, donnés à titre illustratif, à l'appui de la figure annexée :

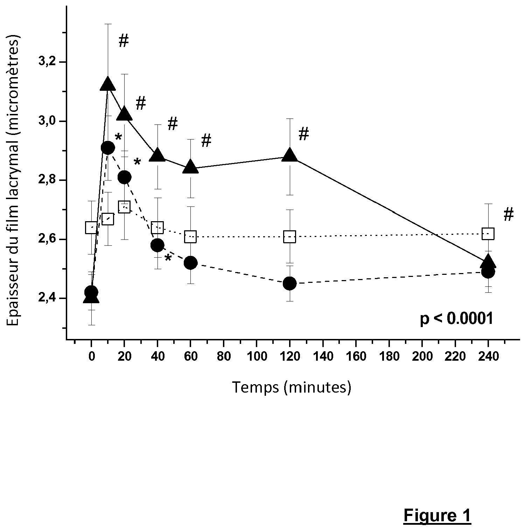

La Figure 1 illustre le suivi, dans le temps, de l'épaisseur du film lacrymal en présence de 3 compositions ophtalmiques topiques, respectivement :
- une composition comprenant une association polymère lubrifiant et oligosaccharide (triangle noir) ;
- une composition comprenant uniquement un polymère lubrifiant (rond noir) ;
- une composition dépourvue de polymère lubrifiant (carré blanc).

### I- Exemple d'une composition selon l'invention

### I-1/Composition :

| **PRODUIT** | **FONCTION** | **FORMULE CENTESIMALE (g/100ml)** |
|---|---|---|
| Hyaluronate de sodium Non réticulé | Polymère lubrifiant et hydratant | 0,15 |
| 100 < MW < 800 kDa | | |
| Rutinose | Oligosaccharide | 3,00 |
| Chlorure de sodium | Agent isotonisant | 0,26 |
| Trometamol | Agent tampon | 0,121 |
| Acide Chlorhydrique concentré | Ajustement du pH | Jusqu'à pH 7,2 |
| Eau | Véhicule | qsp 100 ml |

Après préparation, la composition est avantageusement stérilisée, par exemple sur un filtre stérile à 0,2µm.

Après stérilisation, une telle composition peut être conditionnée dans un flacon unidose (ou usage unique) ou un multidose de type ABAK®. Les unidoses sont classiquement en Polyéthylène basse densité (LDPE) sans additifs.

### I-2/Caractéristiques de la composition :

| **ANALYSE** | **METHODES** | **CARACTERISTIQUES** | |
|---|---|---|---|
| | | **Lot 1** | **Lot 2** |
| **Caractéristiques générales** | | | |
| Apparence (opalescence et couleur) | Current Eur. Ph. (2.2.1.) et (2.2.2, Method II) | Opalesc. ≤ ref. susp. I Colour ≤ ref. sol. B9 | Opalesc. ≤ ref. susp. I Colour ≤ ref. sol. B9 |
| pH | Current Eur. Ph. (2.2.3.) | 6.6 - 7.5 | 6.0 - 7.5 |
| Osmolalité (mosmol/kg) | Current Eur. Ph. (2.2.35.) | 290 - 330 | 170 - 230 |
| Viscosité (mPa.s) | Méthode interne (microviscomètre) | 2.0 - 5.0 | 5.0 - 15.0 |

| **Identification** | | | |
|---|---|---|---|
| Hyaluronate de sodium | A. méthode interne basée sur l'HPLC | Chromatogramme identique à celui du standard | Chromatogramme identique à celui du standard |
| | B. méthode interne basée sur la précipitation | Précipité blanc | Précipité blanc |

| **Test** | | | |
|---|---|---|---|
| Hyaluronate de sodium (g/100 mL) | méthode interne basée sur l'HPLC | 0,1425 - 0,1575 (95 % - 105 %) | 0.285 - 0.315 (95 % - 110 %) |
| **Stérilité** | Current Eur. Ph. (2.6.1.) | Stérile | Stérile |

Il apparaît que la solution obtenue présente les avantages suivants :
- un pH neutre ;
- une formulation hypotonique à isotonique ;
- une viscosité proche des larmes humaines. En effet, la viscosité dans les larmes humaines à 20°C serait d'environ 6,5 mPa.s à gradient de vitesse proche de zéro (+/- 90 mPa.s à 32°C pour le lapin). Or, la force exercée par les paupières lors d'un clignement normale est égale à environ 0,2N et lors d'un clignement forcé environ 0,7 - 0,8N. Lorsque les paupières doivent exercer, lors d'un clignement, une force supérieure ou égale à 0,9N, le patient éprouve une sensation de gêne et d'irritation. Ces sensations sont provoquées par les clignements reflexes qui induisent une élimination rapide grâce aux larmes. Une élimination aisée et non gênante n'est possible que si la viscosité du mélange composition ophtalmique/larmes est inférieure à 50mPa.s, ce qui est le cas en présence d'une composition selon l'invention.

### I-3/ Stabilité de la composition :

La formulation est stable pendant au moins 24 mois lorsqu'elle est conservée à température ambiante.

### I-4/ Toxicité oculaire :

Un test d'irritation oculaire aux expositions répétées a été réalisé pour déterminer le potentiel d'irritation de la composition selon l'invention, suite à des instillations répétées au niveau des tissus oculaires du lapin. Cette étude a été réalisée selon les exigences du standard ISO 10993: Biological Evaluation of Medical Devices, Part 10 (2010): Tests for Irritation and skin sensitization.

Dans les conditions de cette étude, la composition selon l'invention répond aux exigences du test précité.

### I-5/ Sensibilisation par la composition :

Un test a été réalisé pour évaluer le potentiel de la composition selon l'invention à induire une sensibilisation dermique retardée. Cette étude a été réalisée chez le cochon d'Inde en utilisant la méthode de maximisation du standard ISO 10993 et les bonnes pratiques de laboratoire actuellement applicables.

Dans les conditions de cette étude, la composition selon l'invention n'est pas considérée comme sensibilisante.

### II- Etude de l'efficacité d'une composition selon l'invention :

Afin d'évaluer l'efficacité d'une composition selon l'invention similaire à celle de l'exemple I mais dans laquelle l'oligosaccharide est du tréhalose, celle-ci a été comparée à une composition dite standard, à base d'acide hyaluronique, classiquement utilisée dans le traitement de la sécheresse oculaire.

| **PRODUIT** | **FONCTION** | **FORMULE CENTESIMALE (g/100ml)** |
|---|---|---|
| Hyaluronate de sodium Non réticulé | Polymère lubrifiant et hydratant | 0,15 |
| 100 < MW < 800 kDa | | |
| Chlorure de sodium | Agent isotonisant | 0,88 |
| Trometamol | Agent tampon | 0,121 |
| Acide Chlorhydrique concentré (1N) | Ajustement du pH | Jusqu'à pH 7,2 |
| Eau | Véhicule | qsp 100 ml |

But : Le but de la présente étude est de tester l'effet d'une administration unique de 3 substituts lacrymaux différents sur l'épaisseur du film lacrymal chez des patients présentant un syndrome de sécheresse oculaire modéré (DES pour *« Dry Eye Syndrom »*)*.*

Méthode : L'étude est menée selon un schéma aléatoire, en double aveugle et contrôlé avec traitement actif. Des patients avec une DES modérée sont inclus et répartis de manière aléatoire pour recevoir soit des gouttes oculaires contenant une composition selon l'invention non conservée, soit des gouttes non conservées contenant de l'acide hyaluronique seul (voir composition ci-dessus) ou contenant du chlorure de sodium (NaCl à 0,9% ; contrôle) dont la formule est donnée ci-dessous :

| **PRODUIT** | **FORMULE CENTESIMALE (g/100ml)** |
|---|---|
| **Actif:** | |
| Chlorure de Sodium* | 0,9 |

| **Excipients:** | |
|---|---|
| Disodium phosphate dodecahydrate | 0,317 |
| Sodium dihydrogen phosphate dihydrate | 0,067 |
| Eau pour injection | q.s. 100 mL |

L'épaisseur du film lacrymal (TFT pour « *Tear Film Thickness* ») est mesurée par tomographie à cohérence optique de haute résolution (OCT pour « *Optical Coherence Tomography* »). Des mesures sont réalisées avant l'instillation puis 10 minutes, 20 minutes, 40 minutes, 60 minutes (1 heure), 120 minutes (2 heures) et 240 minutes (4 heures) après administration de la composition. De plus, le temps de rupture du film lacrymal (BUT pour « *Break Up Time* »), le test de Schirner et des paramètres subjectifs ont été évalués.

Résultats : Comme il ressort de la figure 1, l'administration du lubrifiant, en l'occurrence de l'acide hyaluronique, augmente significativement la TFT dans le groupe qui a reçu la composition selon l'invention ou la composition à base d'acide hyaluronique seul, alors qu'aucun changement significatif n'est observé après administration de chlorure de sodium. Par ailleurs et pour les 2 groupes où il y a une augmentation, le pic de la TFT est observé 10 minutes après l'administration.

En revanche, l'augmentation de la TFT causée par la combinaison présente dans une composition selon l'invention dure de manière significativement plus longue (jusqu'à 4 heures) que ce qui est observé avec la formulation contenant uniquement l'acide hyaluronique (jusqu'à 40 minutes environ).

Aucune différence significative entre les 3 traitements n'a été observée concernant le BUT et le test de Schirner.

Conclusions : Selon cette étude, une composition selon l'invention s'avère aussi efficace qu'une solution classique à base d'acide hyaluronique et présente une durée d'action significativement prolongée, d'au moins un facteur 5. En pratique et pour un patient, il est possible d'envisager un espacement significatif des instillations pour un même confort. A noter que pour le traitement d'une sécheresse oculaire dite « sévère », la posologie usuelle est une instillation autant de fois que nécessaire, ce qui est très contraignant pour le patient d'où l'utilisation des gels qui sont inconfortable à l'instillation et très difficile à instiller (la formation de la goutte « calibrée » est très difficile). Une composition selon l'invention permet donc de résoudre ce problème.

## Revendications

1. Composition ophtalmique comprenant de l'acide hyaluronique, ou un de ses sels, de poids moléculaire compris entre 100 et 800 kDa et un oligosaccharide.

2. Composition selon la revendication 1, ***caractérisée* en ce que** l'oligosaccharide est un diholoside.

3. Composition selon la revendication 1 ou 2 ***caractérisée* en ce que** l'acide hyaluronique ou un de ses sels représente entre 0,05 et 0,5 g dans 100 ml de la composition, avantageusement entre 0,1 et 0,2 g/100 ml, encore plus avantageusement 0,15 g/100 ml.

4. Composition selon l'une des revendications précédentes ***caractérisée* en ce que** l'oligosaccharide représente entre 0,5 et 5 g dans 100 ml de la composition, avantageusement entre 2 et 4 g/100 ml, encore plus avantageusement 3 g/100 ml.

5. Composition selon l'une des revendications précédentes, ***caractérisée* en ce que** la composition présente une viscosité Brookfield à 25°C inférieure à 50 mPa.s, avantageusement comprise entre 2 et 15 mPa.s.

6. Composition selon l'une des revendications précédentes , ***caractérisée* en ce qu'**elle est dépourvue d'agent conservateur de type antimicrobien.

7. Composition selon l'une des revendications 1 à 6, ***caractérisée* en ce que** la composition ne contient aucun autre actif ophtalmique.

8. Composition selon l'une des revendications 1 à 6, ***caractérisée* en ce que** la composition comprend en outre un actif choisi dans le groupe suivant : aceclidine, acetazolamide, aciclovir, anecortave, apraclonidine, atropine, azapentacene, azelastine, bacitracine, befunolol, betamethasone, betaxolol, bimatoprost, brimonidine, brinzolamide, carbachol, carteolol, celecoxib, chloramphenicol, chlortetracycline, ciprofloxacin, cromoglycate, , cyclopentolate, cyclosporine, dapiprazole, demecarium, dexamethasone, diclofenac, dichlorphenamide, dipivefrine, dorzolamide, echothiophale, emedastine, epinastine, epinephrine, erythromycine, ethoxzolamide, eucatropine, fludrocortisone, fluorometholone, flurbiprofen, fomivirsen, framycetine, ganciclovir, gatifloxacine, gentamycine, homatropine, hydrocortisone, idoxuridine, indomethacine, isoflurophate, ketorolac, ketotifen, latanoprost, levobetaxolol, levobunolol, levocabastine, levofloxacine, lodoxamide, loteprednol, medrysone, methazolamide, metipranolol, moxifloxacine, naphazoline, natamycine, nedocromil, neomycine, norfloxacine, ofloxacine, olopatadine, oxymetazoline, pemirolast, pegaptanib, phenylephrine, physostigmine, pilocarpine, pindolol, pirenoxine, polymyxin B, prednisolone, proparacaine, ranibizumab, rimexolone, scopolamine, sezolamide, squalamine, sulfacetamide, suprofen, tetracaine, tetracyclin, tetrahydrozoline, tetryzoline, timolol, tobramycine, travoprost, triamcinulone, trifluoromethazolarnide, trifluridine, trimethoprime, tropicamide, unoprostone, vidarbine, xylometazoline, des sels pharmaceutiquement acceptables, ou des combinaisons de ceux-ci.

9. Composition selon l'une des revendications précédentes pour son utilisation dans le traitement des pathologies oculaires, notamment de la sécheresse oculaire.

## Patentansprüche

1. Ophthalmische Zusammensetzung mit Hyaluronsäure oder einem ihrer Salze, mit einem Molekulargewicht zwischen 100 und 800 kDa und einem Oligosaccharid.

2. Zusammensetzung gemäß Anspruch 1, ***dadurch gekennzeichnet, dass*** es sich bei dem Oligosaccharid um ein Disaccharid handelt.

3. Zusammensetzung gemäß Anspruch 1 oder 2 ***dadurch gekennzeichnet, dass*** Hyaluronsäure oder eines ihrer Salze zwischen 0,05 und 0,5 g in 100 ml der Zusammensetzung, noch besser zwischen 0,1 und 0,2 g/100 ml, und wiederum besser 0,15 g/100 ml, bilden.

4. Zusammensetzung nach einem der vorhergehenden Ansprüche, ***dadurch gekennzeichnet, dass*** das Oligosaccharid zwischen 0,5 und 5 g in 100 ml der Zusammensetzung, noch besser zwischen 2 und 4 g/100 ml, und wiederum besser 3 g/100 ml, bildet.

5. Zusammensetzung nach einem der vorhergehenden Ansprüche, ***dadurch gekennzeichnet, dass*** die Zusammensetzung eine Brookfield-Viskosität bei 25°C von unter 50 mPa.s, am besten zwischen 2 und 15 mPa.s, aufweist.

6. Zusammensetzung nach einem der vorhergehenden Ansprüche, ***dadurch gekennzeichnet, dass*** sie kein Konservierungsmittel antimikrobiellen Typs enthält.

7. Zusammensetzung gemäß einem der Ansprüche 1 bis 6, ***dadurch gekennzeichnet, dass*** die Zusammensetzung keinen weiteren ophthalmischen Wirkstoff enthält.

8. Zusammensetzung gemäß einem der Ansprüche 1 bis 6, ***dadurch gekennzeichnet, dass*** die Zusammensetzung außerdem einen Wirkstoffe enthält, der aus der folgenden Gruppe ausgewählt wird: Aceclidin, Acetazolamid, Aciclovir, Anecortav, Apraclonidin, Atropin, Azapentacen, Azelastin, Bacitracin, Befunolol, Betamethason, Betaxolol, Bimatoprost, Brimonidin, Brinzolamid, Carbachol, Carteolol, Celecoxib, Chloramphenicol, Chlortetracyclin, Ciprofloxacin, Cromoglycat, Cyclopentolat, Cyclosporin, Dapiprazol, Demecarium, Dexamethason, Diclofenac, Dichlorphenamid, Dipivefrin, Dorzolamid, Echothiophal, Emedastin, Epinastin, Epinephrin, Erythromycin, Ethoxzolamid, Eucatropin, Fludrocortison, Fluorometholon, Flurbiprofen, Fomivirsen, Framycetine, Ganciclovir, Gatifloxacin, Gentamycin, Homatropin, Hydrocortison, Idoxuridin, Indomethacin, Isoflurophat, Ketorolac, Ketotifen, Latanoprost, Levobetaxolol, Levobunolol, Levocabastin, Levofloxacin, Lodoxamid, Loteprednol, Medryson, Methazolamid, Metipranolol, Moxifloxacin, Naphazolin, Natamycin, Nedocromil, Neomycin, Norfloxacin, Ofloxacin, Olopatadin, Oxymetazolin, Pemirolast, Pegaptanib, Phenylephrin, Physostigmin, Pilocarpin, Pindolol, Pirenoxin, Polymyxin B, Prednisolon, Proparacain, Ranibizumab, Rimexolon, Scopolamin, Sezolamid, Squalamin, Sulfacetamid, Suprofen, Tetracain, Tetracyclin, Tetrahydrozolin, Tetryzolin, Timolol, Tobramycin, Travoprost, Triamcinulon, Trifluoromethazolamid, Trifluridin, Trimethoprim, Tropicamid, Unoproston, Vidarbin, Xylometazolin, pharmazeutisch unbedenkliche Salze oder Kombinationen daraus.

9. Zusammensetzung nach einem der vorhergehenden Ansprüche zur Verwendung bei der Behandlung von Augenerkrankungen, insbesondere des trockenen Auges.

## Claims

1. An ophthalmic composition comprising hyaluronic acid, or one of its salts, of a molecular weight comprised between 100 and 800 kDa and an oligosaccharide.

2. Composition according to claim 1, ***characterized in that*** the oligosaccharide is a diholoside.

3. Composition according to claim 1 or 2, ***characterized in that*** the hyaluronic acid or one of its salts constitutes between 0.05 and 0.5 g in 100 ml of the composition, advantageously between 0.1 and 0.2 g/100 ml, even more advantageously 0.15 g/100 ml.

4. Composition according to one of the preceding claims, ***characterized in that*** the oligosaccharide constitutes between 0.5 and 5 g in 100 ml of the composition, advantageously between 2 and 4 g/100 ml, even more advantageously 3 g/100 ml.

5. Composition according to one the preceding claims, ***characterized in that*** the composition displays a Brookfield viscosity at 25 °C lower than 50 mPa.s, advantageously comprised between 2 and 15 mPa.s.

6. Composition according to one the preceding claims, ***characterized in that*** it is deprived of any preservative agent of the anti-microbial type.

7. Composition according to claims 1 to 6, ***characterized in that*** the composition does not contain any other ophthalmic active agent.

8. Composition according to claims 1 to 6, ***characterized in that*** the composition further comprises an active agent chosen among in the following group: aceclidine, acetazolamide, aciclovir, anecortave, apraclonidine, atropine, azapentacene, azelastine, bacitracine, befunolol, betamethasone, betaxolol, bimatoprost, brimonidine, brinzolamide, carbachol, carteolol, celecoxib, chloramphenicol, chlortetracycline, ciprofloxacin, cromoglycate, cyclopentolate, cyclosporine, dapiprazole, demecarium, dexamethasone, diclofenac, dichlorphenamide, dipivefrine, dorzolamide, echothiophale, emedastine, epinastine, epinephrine, erythromycine, ethoxzolamide, eucatropine, fludrocortisone, fluorometholone, flurbiprofen, fomivirsen, framycetine, ganciclovir, gatifloxacine, gentamycine, homatropine, hydrocortisone, idoxuridine, indomethacine, isoflurophate, ketorolac, ketotifen, latanoprost, levobetaxolol, levobunolol, levocabastine, levofloxacine, lodoxamide, loteprednol, medrysone, methazolamide, metipranolol, moxifloxacine, naphazoline, natamycine, nedocromil, neomycine, norfloxacine, ofloxacine, olopatadine, oxymetazoline, pemirolast, pegaptanib, phenylephrine, physostigmine, pilocarpine, pindolol, pirenoxine, polymyxin B, prednisolone, proparacaine, ranibizumab, rimexolone, scopolamine, sezolamide, squalamine, sulfacetamide, suprofen, tetracaine, tetracyclin, tetrahydrozoline, tetryzoline, timolol, tobramycine, travoprost, triamcinulone, trifluoromethazolarnide, trifluridine, trimethoprime, tropicamide, unoprostone, vidarbine, xylometazoline, pharmaceutically approved salts, or any combinations thereof.

9. Composition according to one of the preceding claims for use in the treatment of ocular diseases, such as dry eye.
